# EUROPEAN PATENT APPLICATION

(11) **EP 4 209 505 A1**
(43) Date of publication of application: **12.07.2023**
(21) Application number: 21864234.6
(22) Date of filing: 27.08.2021
(51) Int. Cl.: C07K 14/78, A61K 9/06, A61K 38/16, A61K 47/26, A61P 27/02

(54) **COMPOSITION COMPRISING PEPTIDE, AND USE THEREOF**

(30) Priority: 02.09.2020 JP 2020147265
(71) Applicant: Kola-Gen Pharma Inc., Kyoto-shi, Kyoto 606-8501 (JP)
(72) Inventor: KOJIMA, Kentaro, Kyoto-shi, Kyoto 604-0941 (JP); TANAKA, Hiroshi, Kyoto-shi, Kyoto 606-0855 (JP); SHINKAI, Yoichiro, Kyoto-shi, Kyoto 602-0894 (JP); KOIDE, Takaki, Asaka City, Saitama 3510014 (JP); ICHISE, Shinichiro, Tokyo 177-0054 (JP); NOSE, Hiroshi, Kyoto-shi, Kyoto 615-8245 (JP)
(74) Representative: Lederer & Keller Patentanwälte Partnerschaft mbB
(86) International application number: PCT/JP2021/031436
(87) International publication number: WO 2022/050176

(57) **Abstract**

The present invention provides a composition comprising a novel peptide, and a use thereof (for example, the treatment of a macular hole).

## Description

### TECHNICAL FIELD

The present invention relates to a novel composition comprising peptides and use thereof.

### BACKGROUND TECHNOLOGY

The macula lutea is the part of retina that mostly relates to visual acuity. A disease in which a hole is formed in the macular lutea is referred to as macular hole, which makes it difficult to see things. The macular hole is caused by aging of eyes, in particularly changes of vitreous bodies due to aging. Since the retina and the vitreous cortex are strongly bonded, it is considered that the atrophy of vitreous collagen fiber due to aging applies tension to the retina, and the traction force to the front consequently forms cracks in the retina and becomes a hole in macula lutea. Since the disease is predominant in the elderly, the number of its patients is increasing along with the rapid increase in elderly population in recent years.

The only treatment for macular hole is vitreous surgery, which closes the circular hole by removing the causative vitreous. If the symptoms of macular hole are mild and the circular hole is small, the closure rate is high. However, if the symptoms progress and the circular hole is large, the closure rate of the circular hole is poor and recovery of visual acuity cannot be expected. The recovery rate of visual acuity after the vitreous surgery is 70% and hospitalization for 3 to 7 days is necessary. In addition, in the treatment of macular hole, although the macular hole can be closed, there is no scaffold for the reconstruction of macular cells after the surgery, because the endoscopy membrane, which is the basement membrane, is removed. It remains a problem that the quality of healing is not guaranteed. As described above, development of an effective therapeutic agent for macular hole is desired.

### SUMMARY OF THE INVENTION

### PROBLEMS TO BE SOLVED BY THE INVENTION

In such circumstances, it is an object of the present invention to provide a novel therapeutic agent for macular hole, aiming at the recovery of quality of vision (QOV) and the improvement of quality of life (QOL) after the vitreous surgery for macular hole.

### MEANS FOR SOLVING THE PROBLEMS

The present inventors conducted intensive studies on artificial collagen-like polypeptide membrane. As a result, it has been found out that a scaffold necessary for the regeneration of retinal cells can be provided by using a special composition comprising peptides, and circular holes can be closed just by sticking to recover and improve visual acuity and reduce the burden on patients. The present inventors further continued the studies and completed the present invention.

That is, the present invention relates to the following matters.
(1) A composition comprising two or more polymerized peptides having polymerization units of three-stranded peptides represented by formula (I)
   Formula (I):

   - (P₁, P₂, P₃) - (I)

   (wherein,
   P₁, P₂, and P₃ may be the same or different and are each independently a peptide chain represented by the following formula (II),
   P₁, P₂, and P₃ form a trimer having a triple helix structure, and are polymerized by oxidative crosslinking with disulfide bonds by cysteine (Cys) residues contained in each peptide chain,
      Formula (II):

      R¹-(Pro-Hyp-Gly) m-X-(Pro-Hyp-Gly) n-R² (II)

      (wherein,
      R¹ and R² each have an amino terminal and a carboxy terminal, and are each independently a peptide group consisting of any 2 to 10 amino acid residues comprising at least two Cys (cysteine) residues,
      Pro is a proline residue,
      Hyp is a hydroxyproline residue,
      Gly is a glycine residue,
      m and n are each independently an integer of 4 or more,
      X is a peptide group represented by 1 to 4 repeated -Xaa-Yaa-Gly-, and
      Xaa and Yaa each independently represent an amino acid residue.)).
(2) The composition according to (1), wherein the P₁, P₂, and P₃ are the same peptide chains.
(3) The composition according to (1), wherein the X is a peptide group consisting of an amino acid residue selected from -Phe-Hyp-Gly-Glu-Arg-Gly-, Pro-Arg-Gly-Gln-Hyp-Gly-Val-Met-Gly-Phe-Hyp-Gly-, and -Pro-Lys-Gly-His-Arg-Gly-Phe-Ser-Gly-Leu-Hyp-Gly-.
(4) The composition according to (1), wherein the X is a peptide group consisting of an amino acid residue selected from -Pro-Arg-Gly- or -Phe-Hyp-Gly-Glu-Arg-Gly-.
(5) The composition according to (1), wherein the R¹ and R² are each independently a peptide group consisting of an amino acid residue of two or more Cys (cysteine) residues.
(6) The composition according to (1), wherein the peptide chain represented by formula (II) is selected from

   R¹- (Pro-Hyp-Gly) m-Pro-Arg-Gly- (Pro-Hyp-Gly) n-R² (i)

   and

   R¹- (Pro-Hyp-Gly) m-Phe-Hyp-Gly-Glu-Arg-Gly- (Pro-Hyp-Gly) n-R² (ii)
(7) The composition according to (1), wherein the peptide chain represented by formula (II) is indicated by the following SEQ ID NOs.
   H-Cys-Cys-Cys-(Pro-Hyp-Gly)5-Pro-Arg-Gly-(Pro-Hyp-Gly)4-Cys-Cys-Cys-OH SEQ ID NO: 1
   H-Cys-Cys-(Pro-Hyp-Gly) 5-Pro-Arg-Gly-(Pro-Hyp-Gly) 4-Cys-Cys-OH SEQ ID NO: 2
   H-Cys-Cys-Cys-(Pro-Hyp-Gly)4-Phe-Hyp-Gly-Glu-Arg-Gly-(Pro-Hyp-Gly)4-Cys-Cys-Cys-OH
      SEQ ID NO: 3
(8) The composition according to any one of (1) to (7), further comprising an aqueous filler.
(9) The composition according to (8), wherein the aqueous filler is a sugar.
(10) The composition according to (9), wherein the sugar is one or more of glucose, lactose, sucrose, mannitol, trehalose, and sorbitol.
(11) The composition according to (10), wherein the sugar is glucose.
(12) The composition according to any one of (8) to (11), wherein the aqueous filler is present in an amount of 10 to 50% relative to the total weight of the composition.
(13) The composition according to any one of (1) to (12), wherein the polymerized peptide is present in an amount of 50 to 90% relative to the total weight of the composition.
(14) The composition according to any one of (1) to (13), which is in a form of gel.
(15) The composition according to any one of (1) to (13), which is in a transparent form.
(16) A pharmaceutical product comprising the composition according to any one of (1) to (15).
(17) A device comprising the composition according to any one of (1) to (15).
(18) A therapeutic agent for an eye disease comprising the composition according to any one of (1) to (15).
(19) The therapeutic agent for an eye disease according to (18), wherein the eye disease is macular hole or retinal detachment.
(20) A treatment method for an eye disease, comprising a step of administering to a subject a therapeutically effective amount of the composition according to any one of (1) to (15).
(21) The treatment method according to (20), wherein the eye disease is macular hole or retinal detachment.
(22) A production method of the composition according to any one of (1) to (15), which comprises making the polymerized peptide represented by the formula (I) and the aqueous filler into a homogeneous physical mixture.
(23) Use of the composition according to any one of (1) to (15) in the production of the pharmaceutical product for treating an eye disease.

### EFFECTS OF THE INVENTION

The peptide membrane consisting of the composition of the present invention has high transparency and is ideal for treatment of macular hole.

According to the present invention, it is possible to process the peptide membrane into a sheet-like shape having an optimum thickness while maintaining its strength.

According to the present invention, it is possible to impart a specific physiological function and adjust a combination of its strength.

According to the present invention, it is possible to control the adhesion, proliferation, differentiation, and the like of cells by changing the stiffness of the scaffold.

According to the present invention, it is possible to control the decomposition speed of the peptide membrane.

According to the present invention, it is possible to industrially produce the peptide membrane.

### BRIEF DESCRIPTION OF THE DRAWINGS

Figure 1 shows an application test of a peptide membrane of the present invention to sites where MIO-M1 cells are deficient.
Figure 2 shows that MIO-M1 cells are binding and proliferating on a peptide membrane of the present invention.
Figure 3 shows verification (*in vivo*) of efficacy and safety with a white rabbit model using a peptide membrane not added with an aqueous filler.
Figure 4 shows verification (*in vivo*) of efficacy and safety with a white rabbit model using a peptide membrane added with an aqueous filler.
Figure 5 shows verification (*in vivo*) of efficacy and safety with a cynomolgus model.

### EMBODIMENTS FOR CARRING OUT THE INVENTION

The composition of the present invention comprises two or more kinds of polymerized peptides.

### POLYMERIZED PEPTIDES (THE FIRST COMPONENT)

The polymerized peptides of the present invention are those having polymerization units of three-stranded peptides represented by formula (I):

- (P₁, P₂, P₃) - (I)

(wherein,
P₁, P₂, and P₃ may be the same or different and are each independently a peptide chain represented by the following formula (II),
P₁, P₂, and P₃ form a trimer having a triple helix structure, and are polymerized by oxidative crosslinking with disulfide bonds by cysteine (Cys) residues contained in each peptide chain,
   Formula (II):

   R¹-(Pro-Hyp-Gly) m-X-(Pro-Hyp-Gly) n-R² (II)

   (wherein,
   R¹ and R² each have an amino terminal and a carboxy terminal, and are each independently a peptide group consisting of any 2 to 10 amino acid residues comprising at least two Cys (cysteine) residues,
   m and n are each independently an integer of 4 or more, and
   X is a peptide group represented by 1 to 4 repeated -Xaa-Yaa-Gly-).).

The polymerized peptides of the present invention are characterized by having structural units of trimer peptides formed from three peptide chains represented by formula (I) below and being oxidative crosslinked.

- (P₁, P₂, and P₃) - (I)

(wherein, P₁, P₂, and P₃ may be the same or different and are each independently a peptide chain represented by formula (II) below. P₁, P₂, and P₃ may form a trimer having a triple helix structure, and may be crosslinked with disulfide bonds by cysteine (Cys) residues contained in each peptide chain. The trimer is polymerized by oxidative crosslinking with disulfide bonds of the Cys.

R¹-(Pro-Hyp-Gly) m-X-(Pro-Hyp-Gly) n-R² (II)

(wherein, R¹ and R² are each an amino terminal and a carboxy terminal, and are each independently a peptide group consisting of any 2 to 10 amino acid residues comprising at least two Cys residues. X is a peptide group represented by 1 to 4 repeated -(Xaa-Yaa-Gly)-. m and n are each independently an integer of 4 or more.)

The Xaa and Yaa each independently represent an amino acid residue, for example, selected from proline (Pro or P) residue, hydroxyproline (Hyp or O) residue, arginine (Arg or R) residue, lysine (Lys or K) residue, valine (Val or V) residue, leucine (Leu or L) residue, isoleucine (Ile or I) residue, serine (Ser or S) residue, threonine (Thr or T) residue, alanine (Ala or A) residue, glycine (Gly or G) residue, phenylalanine (Phe or F) residue, methionine (Met or M) residue, glutamic acid (Glu or E) residue, aspartic acid (Asp or D) residue, asparagine (Asn or N) residue, glutamine (Gln or Q) residue, histidine (His or H) residue, tryptophan (Trp or W) residue, or tyrosine (Tyr or Y) residue. The proline residue may be modified with an amino group or a fluorine atom. An N-isobutyl group glycine residue may be used in the positions of Xaa and Yaa.

The number of Cys residues contained within 10 residues from the N-terminal and C-terminal of the peptide chains may independently be the same or different, or may be two or more, three or more, four or more, or five or more.

In the structure of the polymerized peptides, the peptide groups from each of the amino terminal and the carboxy terminal comprising at least two cysteine (Cys) residues may be within 10 residues, and additionally within 9, 8, 7, 6, 5, 4, or 3 residues.

In the present specification, the term "polymerized peptide" refers to a peptide polymerized through a disulfide bond formed by oxidative crosslinking between cysteine residues contained in the peptides.

The degree of polymerization of the polymerized peptides of the present invention is 2 or more, and is not particularly limited as long as a gel, preferably a hydrogel, comprising the polymerized peptides of the present invention can be formed. The average degree of polymerization may be less than 100, 100 to 500, 500 to 1000, 1000 to 5000, 5000 to 10000, or 10000 or more.

In the present specification and the sequence table attached thereto, the structures of peptides are described by the notation methods of three characters or one character of amino acids known to those of ordinary skilled in the art. In the present specification, the amino acids are in L-forms. The amino acid of the present specification comprises modified amino acid residues known in the art, for example, 4-hydroxy-L-proline, 4-fluoro-L-proline, and N-isobutyl group glycine, in addition to the 20 kinds of L-amino acids known to be used for translation of general proteins in molecular biology. In the present specification, hydroxyproline is 3-hydroxyproline or 4-hydroxy-L-proline, and is represented by "Hyp" in the three-character notation and "O" in the one-character notation.

The polymerized peptides according to the present invention can be produced by, but is not limited to, a well-known chemically synthetic method of peptides using commercially available amino acids (Patent No. 6455862). In addition, a nucleic acid sequence encoding a desired amino acid sequence is produced and incorporated into an expression vector to generate a recombinant expression vector by a well-known method, and generate a transformant by introducing into a suitable host such as a microorganism like Escherichia coli. Since the gene recombinant peptide chains are generated by culturing the obtained transformant in a suitable culture medium, the gene recombinant peptide chains used in the present invention can be prepared by recovering the gene recombinant peptide chains generated from the culture.

Such peptide chains can be obtained from separation and purification by means of high-performance liquid chromatography, etc. and can be used in the production of three-stranded peptides.

In the present invention, polymerized peptides in which the peptides are crosslinked with disulfide bonds are provided by producing the polymerized peptides in which a plurality of cysteine residues are incorporated into the peptide chains and crosslinking oxidatively, but are not limited thereto, with an oxidizing agent such as dimethyl sulfoxide (DMSO) or air oxidation.

Examples of the oxidizing agent for producing the polymerized peptides of the present invention include DMSO as described above and additionally oxygen, iodine, hydrogen peroxide, sodium bromate (sodium bromate), potassium bromate, sodium perborate, or potassium perchlorate. However, they are not limited thereto.

The formation of the crosslinking with disulfide bonds can be confirmed by, for example, using Ellman's reagent to quantify the remaining thiol group after oxidative crosslinking reaction.

Furthermore, a thin film of polymerized peptide having improved strength as described below is provided by drying a hydrogel consisting of polymerized peptides produced in an aqueous solvent in the present invention.

The composition of the present invention comprises two or more kinds of different polymerized peptides. Different polymerized peptides can be selected depending on the physiological active strength and physical strength of the thin film of polymerized peptide. The proportion of different polymerized peptides in the composition can be adjusted. For example, a film can be made using three kinds of the following peptides in proportions of 5: 5: 1, 5: 4: 1, or 5: 3: 1.
C2s: CCPO (GPO)₄GPR (GPO)₄GCC
   H-Cys-Cys-(Pro-Hyp-Gly)5-Pro-Arg-Gly-(Pro-Hyp-Gly)4-Cys-Cys-OH SEQ ID NO: 2
C3s: CCCPO (GPO) ₄GPR (GPO) ₄GCCC
   H-Cys-Cys-Cys-(Pro-Hyp-Gly)5-Pro-Arg-Gly-(Pro-Hyp-Gly)4-Cys-Cys-Cys-OH SEQ ID NO: 1
C3s-GFOGER : CCCPO(GPO)₃GFOGER(GPO)₄GCCC

H-Cys-Cys-Cys-(Pro-Hyp-Gly)4-Phe-Hyp-Gly-Glu-Arg-Gly-(Pro-Hyp-Gly)4-Cys-Cys-
Cys-OH    SEQ ID NO: 3

When the number of cysteine is large, a stiff membrane is formed, and when the number of cysteine is small, a soft membrane is formed. Therefore, the physical strength is determined by the number of cysteine.

On the other hand, GFOGER is an integrin-binding sequence that allows cells to adhere. Therefore, by appropriately mixing C3s-GFOGER, the physiological active strength is increased.

The content (%) of the "polymerized peptides" in the whole composition (comprising a polymerized peptide and an aqueous filler) according to the present invention is preferably 50 to 90% ("%" indicates weight percent unless otherwise noted in the present specification).

### AQUEOUS FILLERS (THE SECOND COMPONENT)

The composition of the present invention may comprise an aqueous filler in addition to polymerized peptides. Examples of the aqueous filler include, but are not limited to, potassium carbonate, sodium carbonate, ammonium carbonate, calcium lactate, mannitol, urea, inositol, magnesium succinate, sorbitol, and carbohydrates (for example, one of sugars such as glucose, lactose, sucrose, mannitol, trehalose, and sorbitol), or a combination thereof. However, glucose is preferable.

The content (%) of the "aqueous filler" in the whole composition (comprising a polymerized peptide and an aqueous filler) according to the present invention is preferably 10 to 50%.

The composition of the present invention can comprise an excipient in addition to the polymerized peptides and the aqueous filler.

Examples of the excipient include mannitol, croscarmellose sodium, hydroxypropyl cellulose, hydroxypropyl methylcellulose, polyethylene glycol, polyvinylpyrrolidone, crystalline cellulose, lactose, white sugar, starch, corn starch, titanium oxide (TiO2), light anhydrous silicic acid, and the like. These excipients may be used alone or in combination of two or more thereof.

### A METHOD FOR PRODUCING A PEPTIDE MEMBRANE

Hydrogel of peptide membrane can be produced by dissolving different polymerized peptides in solvents respectively and then gelling.

In addition, hydrogel of peptide membrane can be produced by dissolving different polymerized peptides in solvents respectively, then adding an aqueous filler, and gelling.

In addition, the hydrogel can be dried to produce a sheet-like thin film of polymerized peptide. The thin film of polymerized peptide can be used, for example, as a therapeutic material for macular hole.

By drying the thin film of polymerized peptide of the present invention, it can be stored at room temperature for a longer period as compared with the hydrogel consisting of the polymerized peptide before being dried and is imparted with better strength even after rehydration as compared with the original hydrogel, since the interaction between peptide molecules changes. Therefore, the thin film of polymerized peptide of the present invention has improved strength and can be used as a heat-resistant medical material that can be sterilized by heating.

In the present specification, the term "stiffness of gel" refers to, for example, the robustness of gel against decomposition *in vivo* by phagocytic cells, such as macrophages. The stiffness of gel is affected by the degree of crosslinking polymerization of the gel or the difference in the branched structure of the crosslinked peptide chains or the like. For example, if a large amount of crosslinking with disulfide bonds is contained in gel, a stiff gel is formed.

The composition of the present invention may be used as it is as a therapeutic agent for macular hole or retinal detachment, or may be formulated into various dosage forms by methods well-known to those skilled in the art using a pharmaceutically acceptable carrier or excipient. The carrier or excipient to be used is well-known to those skilled in the art and can be appropriately selected. The drug of the present invention can be produced by means and methods well-known to those skilled in the art. For example, in the case of producing an agent for injection or infusion, a pharmaceutically acceptable carrier such as physiological saline or phosphate buffered physiological saline can be used. When preparing the drug of the present invention, a pharmaceutically acceptable additive such as thickener, absorption enhancer, pH adjuster, preservative, dispersant, wetting agent, stabilizer, antiseptic agent, suspension, surfactant, or the like may be used.

The dosage form of the drug of the present invention is not particularly limited, and can be appropriately selected according to the region, size, and type of the macular hole or retinal detachment to be treated and conditions of patients, or the like. The drug of the present invention may be a liquid, semi-solid or solid. Examples of the dosage form of the drug of the present invention include, but are not limited thereto, thin film. Alternatively, the drug of the present invention may be in the form of a freeze-dried product which is suspended in a pharmaceutically acceptable carrier such as physiological saline or phosphate buffered physiological saline at the time of administration.

The administration route of the drug of the present invention is not particularly limited either, and can be appropriately selected according to the region, size, and type of the macular hole or retinal detachment to be treated and conditions of patients, or the like. Examples of the administration route of the drug of the present invention include, but are not limited to, placement by injection or incision and the like.

The dose of the drug of the present invention can be appropriately determined by a doctor according to the region, size, and type of the macular hole or retinal detachment to be treated and conditions of patients, or the like.

The drug of the present invention can be administered one or more times. The number of administrations can be determined by a doctor in consideration of the region, type, and degree of macular hole and conditions of patients, or the like

In yet another embodiment, the present invention provides use of the composition for producing a therapeutic agent for macular hole or retinal detachment.

In yet another embodiment, the present invention provides use of the composition for the treatment of macular hole or retinal detachment.

In yet another embodiment, the present invention provides a method for treating macular hole, which comprises the administration of the composition to a patient suffering from macular hole or retinal detachment.

Hereafter, the present invention will be described in more detail and specifically with reference to examples. However, the examples are not intended to limit the scope of the present invention.

### EXAMPLES

Hereafter, the abbreviations used in the examples will be described.
AMINO ACID RESIDUES (All of which are in L forms.)
Arg (R): arginine
Asp (D): aspartic acid
Cys (C): cysteine
Gln (Q): glutamine
Glu (E): glutamic acid
Gly (G): glycine
His (H): histidine
Hyp (O): 4-hydroxyproline
Lys (K): lysine
Pro (P): proline
Tyr (Y): tyrosine
Ile (I): isoleucine
Leu (L): leucine
Met (M): methionine
Phe (F): phenylalanine
Ser (S): serine
Val (V): valine

### PROTECTIVE GROUPS

Fmoc: 9-fluorenylmethoxycarbonyl
tBu: tert-butyl
Trt: triphenylmethyl (trityl)
Pbf: 2,2,4,6,7-pentamethyldihydrobenzofuran-5-sulfonyl
Boc: tert-butoxycarbonyl
Resin CTC: 2-chlorotrityl chloride

### REAGENTS

BCA: bicinchoninic acid
BSA: bovine serum albumin
CHCA: alpha-cyano-4-hydroxycinnamic acid
DCM: dichloromethane
DIC: N, N'-diisopropylcarbodiimide
DIEA: N, N-diisopropylethylamine
DMAP: N, N-dimethylaminopyridine
DMF: N, N-dimethylformamide
DMSO: dimethyl sulfoxide
DTNB: 5,5'-dithiobis-2-nitrobenzoic acid
DTT: dithiothreitol
EDT: ethanedithiol
EDTA: ethylenediaminetetraacetic acid
FBS: fetal bovine serum
HOBt: 1-hydroxybenzotriazole
MeCN: acetonitrile
MeOH: methanol
MOPS: 3-morpholinopropanesulfonic acid
NEM: N-ethylmaleimide
PBS: phosphate buffer
PMSF: phenylmethylsulfonyl fluoride
SDS: sodium dodecyl sulfate
TBS: Tris-buffered saline
TFA: trifluoroacetic acid
TMSO: tetramethylene sulfoxide

### EQUIPMENTS

RP-HPLC: reverse-phase high-performance liquid chromatography
MALDI-TOF-MS: matrix-assisted laser desorption / ionization time-of-flight mass spectrometer

### OTHERS

CD: circular polarization dichroism
DDR: discoidin domain receptor
ECM: extracellular matrix
FAK: focal adhesion kinase
HDF: human dermal fibroblast
HRP: horseradish peroxidase
HSPG: heparan sulfate proteoglycan
PEDF: pigment epithelium-derived factor
SDS-PAGE: sodium dodecyl sulfate-polyacrylamide gel electrophoresis
vWF: von Willebrand factor

The examples of the present invention described below are merely illustrative and are not intended to limit the technical scope of the present invention. The technical scope of the present invention is limited only by the description of the claims. Modifications of the present invention, for example, addition, deletion, and replacement of the constituent elements of the present invention may be made provided that they do not deviate from the gist of the present invention.

### EXAMPLE 1

### A METHOD FOR PRODUCING A PEPTIDE MEMBRANE

Each of the peptide powders C2s, C3s, and C3s-GFOGER is weighed and dissolved in a degassed 0.05% TFA solution to make a solution of 12.5 mg/mL.

After heating at 85°C for 5 minutes, it is left at room temperature for a while, and let stand at 4°C for one day. The solutions of C2s, C3s, and C3s-GFOGER are mixed at a ratio of 5: 4: 1, and DMSO is added and mixed to make a final concentration of 10%. The mixed solution is injected onto an acrylic plate serving as a template, and let stand at room temperature for 3 days in a sealed container under a wet condition.

In case of addition of an aqueous filler, after heating at 85°C for 5 minutes, it is left at room temperature for a while, and let stand at 4°C for one day. The solutions of C2s, C3s, and C3s-GFOGER, 10%(w/v) glucose solution, and DMSO are mixed at a ratio of 5: 4: 1: 1. The mixed solution is injected onto an acrylic plate serving as a template, and let stand at room temperature for 3 days in a sealed container under a wet condition.

After 3 days, the whole acrylic plate is immersed in pure water so that the gel does not collapse, and let stand at room temperature for 1 to 2 hours.

Pure water is removed, the whole acrylic plate of the template is placed in a drying machine and dried overnight.

After drying, the peptide membrane is punched out with a trepan having a diameter of 2 mm, and the peptide membrane is subjected to a test.

### TEST EXAMPLE 1

### APPLICATION TEST OF A PEPTIDE MEMBRANE ADDED WITH AN AQUEOUS FILLER TO SITES WHERE MIO-M1 CELLS ARE DEFICIENT

### Test Method:

MIO-M1 strain of human Muller cells is seeded on a 6 well-Transwell plate and cultured in a CO₂ incubator. When the cells become confluent, the insert is taken out of the plate and transferred to a 60 mm-dish.

The culture solution in the insert is sucked and removed. The center of the bottom surface is punched out with a disposable trepan for biopsy having a diameter of 1 mm. The perforation is confirmed.

The insert is transferred to a 6 well-Transwell plate containing a culture medium. The amount of medium is set so that the bottom surface of the insert to which the cells are attached is barely immersed. The peptide membrane is placed to cover the central perforated portion of the insert.

A small amount of the medium is added to the bottom surface of the insert and transferred into the CO₂ incubator and cultured. Observation is carried out over time under a microscope to confirm that MIO-M1 cells are adhering to the peptide membrane and proliferating.

### Results:

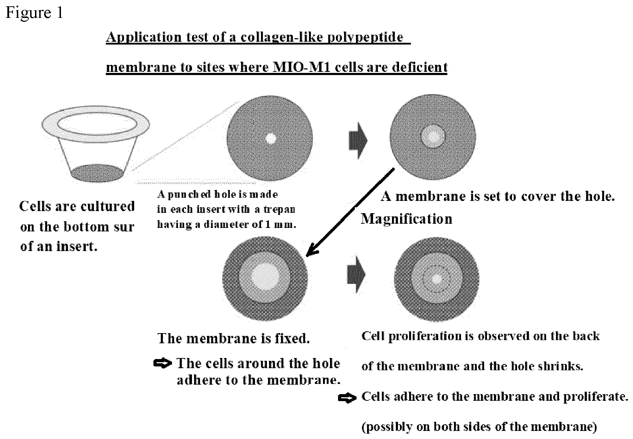

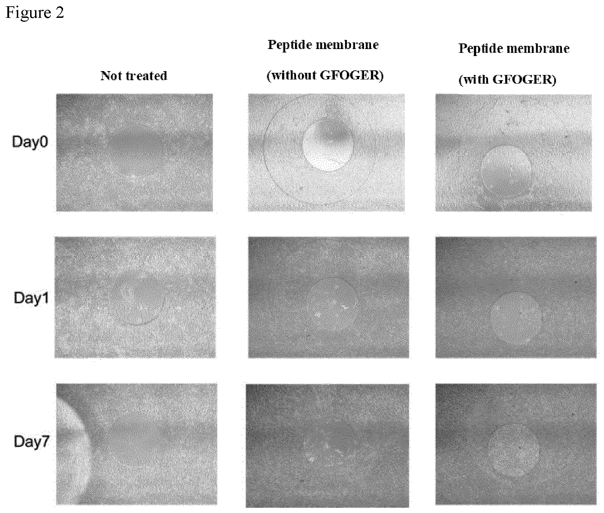

In the group in which the perforated hole of 1 mm made by a disposable trepan for biopsy is covered with a peptide membrane (without GFOGER), cell elongation or proliferation is not observed, since the MIO-M1 strain of human Muller cells cannot adhere to the peptide membrane. On the other hand, in the group in which the perforated hole is covered with a peptide membrane (with GFOGER), cell elongation or proliferation is observed, since the MIO-M1 strain of human Muller cells adheres to the peptide membrane (Figures 1 and 2).

### TEST EXAMPLE 2

### VERIFICATION (IN VIVO) OF EFFICACY AND SAFETY WITH A WHITE RABBITS MODEL

### Test Method:

A rabbit is anesthetized (ketamine + xylazine) and an implantation test is carried out. A surface anesthetic agent (0.4% benoxil ophthalmic solution, Santen Pharmaceutical Co., Ltd.) is applied to the right eye. The animal is held in supine position. The right eye is opened with a speculum. The eyeball is disinfected and washed with Isodine solution diluted 40-fold with physiological saline or PA / iodine eye drops / eyewash diluted 4-fold with physiological saline and rinsed with physiological saline. The surgical site is covered with a protective sheet. After the vitreous body is excised by vitreous surgery, a circular hole of about 500 µm is made. Thereafter, a peptide membrane and a peptide membrane added with an aqueous filler are inserted into the eye from the sclera, and indwelled in the circular hole. The test substance is fixed by using a combination of a viscoelastic substance, nitrogen gas replacement, perfluorocarbon, or the like. Optical coherence tomography (OCT) is performed at the completion of the implantation, as well as 2 days and one week later, and the adhesion is assessed.

### Results:

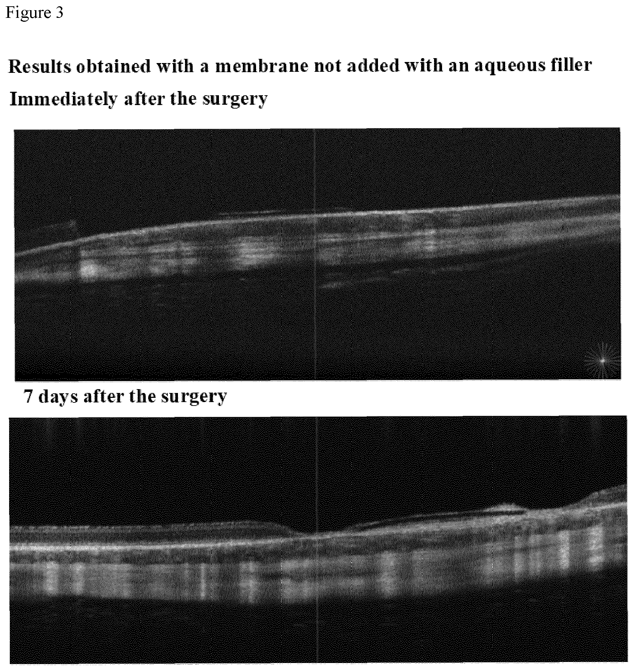

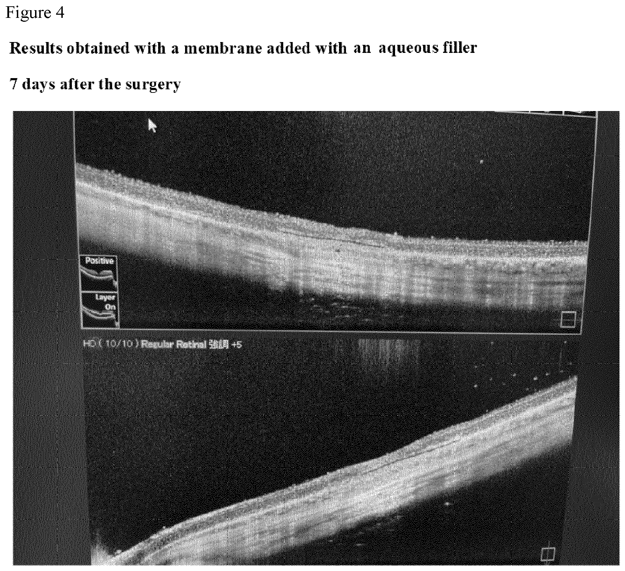

In the case of combined use of viscoelastic substances, adhesion was observed after 1 week and no inflammatory site or the like was observed (Figure 3: a peptide membrane not added with an aqueous filler; and Figure 4: a peptide membrane added with an aqueous filler).

### TEST EXAMPLE 3

### VERIFICATION (IN VIVO) OF EFFICACY AND SAFETY WITH A CYNOMOLGUS MODEL

### Test Method:

A male cynomolgus is anesthetized and an implantation test is carried out. The skin around the eyelid is disinfected with cotton or sterilized gauze immersed in 10% Isodine solution (Meiji Seika Pharma Co., Ltd.), or the like. After drying, the eyelid is disinfected with cotton immersed in PA / iodine eye drops / eyewash solution (ROHTO Pharmaceutical Co., Ltd.) diluted 4-fold with physiological saline (Otsuka Pharmaceutical Co., Ltd.). A surface anesthetic (0.4% benoxil ophthalmic solution, Santen Pharmaceutical Co., Ltd.) is applied to the right eye. The animal is held in supine position. The right eye is opened with a sterilized speculum. The eyeball is disinfected and washed with Isodine solution diluted 40-fold with physiological saline or PA / iodine eye drops / eyewash diluted 4-fold with physiological saline and rinsed with physiological saline. The surgical site is covered with a protective sheet. The vitreous body is excised by vitreous surgery, and a vitreous body forceps are inserted to partially separate the inner boundary membrane (about 4 mm in diameter) of the site corresponding to macula lutea. After a circular hole (0.5 to 1 optic disc diameter) in the macula lutea is made, a peptide membrane added with an aqueous filler is inserted into the eye and indwelled in the circular hole. Gas replacement is performed in the eye as necessary, and the test substance is pressure-bonded to the retina. After the implantation, the model preparation and implantation site of the test substance are recorded based on the macula lutea.

### Results:

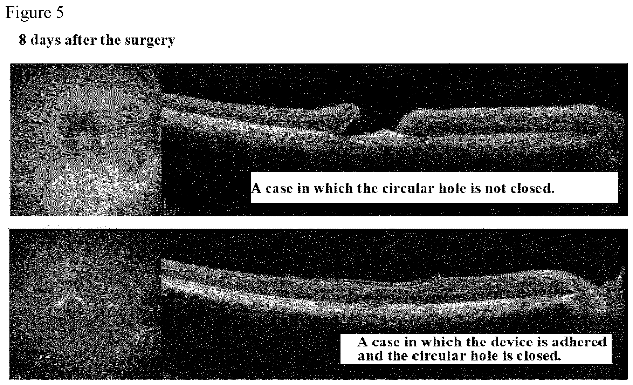

In the cases of intraoperative or postoperative gas replacement, adhesion was observed after 8 days and no inflammation site or the like was observed (Figure 5).

## Claims

1. A composition comprising two or more polymerized peptides having polymerization units of three-stranded peptides represented by formula (I)
Formula (I):
- (P₁, P₂, P₃) - (I)
(wherein,
P₁, P₂, and P₃ may be the same or different and are each independently a peptide chain represented by the following formula (II),
P₁, P₂, and P₃ form a trimer having a triple helix structure, and are polymerized by oxidative crosslinking with disulfide bonds by cysteine (Cys) residues contained in each peptide chain,
Formula (II):
R¹-(Pro-Hyp-Gly) m-X-(Pro-Hyp-Gly) n-R² (II)
(wherein,
R¹ and R² each have an amino terminal and a carboxy terminal, and are each independently a peptide group consisting of any 2 to 10 amino acid residues comprising at least two Cys (cysteine) residues,
Pro is a proline residue,
Hyp is a hydroxyproline residue,
Gly is a glycine residue,
m and n are each independently an integer of 4 or more,
X is a peptide group represented by 1 to 4 repeated -Xaa-Yaa-Gly-, and
Xaa and Yaa each independently represent an amino acid residue.)).

2. The composition according to claim 1, wherein the P₁, P₂, and P₃ are the same peptide chains.

3. The composition according to claim 1, wherein the X is a peptide group consisting of an amino acid residue selected from -Phe-Hyp-Gly-Glu-Arg-Gly-, Pro-Arg-Gly-Gln-Hyp-Gly-Val-Met-Gly-Phe-Hyp-Gly-, and -Pro-Lys-Gly-His-Arg-Gly-Phe-Ser-Gly-Leu-Hyp-Gly-.

4. The composition according to claim 1, wherein the X is a peptide group consisting of an amino acid residue selected from -Pro-Arg-Gly- or -Phe-Hyp-Gly-Glu-Arg-Gly-.

5. The composition according to claim 1, wherein the R¹ and R² are each independently a peptide group consisting of an amino acid residue of two or more Cys (cysteine) residues.

6. The composition according to claim 1, wherein the peptide chain represented by formula (II) is selected from
R¹- (Pro-Hyp-Gly) m-Pro-Arg-Gly- (Pro-Hyp-Gly) n-R² (i)
and
R¹- (Pro-Hyp-Gly) m-Phe-Hyp-Gly-Glu-Arg-Gly- (Pro-Hyp-Gly) n-R² (ii).

7. The composition according to claim 1, wherein the peptide chain represented by formula (II) is indicated by the following SEQ ID NOs.
H-Cys-Cys-Cys-(Pro-Hyp-Gly)5-Pro-Arg-Gly-(Pro-Hyp-Gly)4-Cys-Cys-Cys-OH SEQ ID NO: 1
H-Cys-Cys-(Pro-Hyp-Gly) 5-Pro-Arg-Gly-(Pro-Hyp-Gly) 4-Cys-Cys-OH SEQ ID NO: 2
H-Cys-Cys-Cys-(Pro-Hyp-Gly)4-Phe-Hyp-Gly-Glu-Arg-Gly-(Pro-Hyp-Gly)4-Cys-Cys-Cys-OH
SEQ ID NO: 3

8. The composition according to any one of claims 1 to 7, further comprising an aqueous filler.

9. The composition according to claim 8, wherein the aqueous filler is a sugar.

10. The composition according to claim 9, wherein the sugar is one or more of glucose, lactose, sucrose, mannitol, trehalose, and sorbitol.

11. The composition according to claim 10, wherein the sugar is glucose.

12. The composition according to any one of claims 8 to 11, wherein the aqueous filler is present in an amount of 10 to 50% relative to the total weight of the composition.

13. The composition according to any one of claims 1 to 12, wherein the polymerized peptide is present in an amount of 50 to 90% relative to the total weight of the composition.

14. The composition according to any one of claims 1 to 13, which is in a form of gel.

15. The composition according to any one of claims 1 to 13, which is in a transparent form.

16. A pharmaceutical product comprising the composition according to any one of claims 1 to 15.

17. A device comprising the composition according to any one of claims 1 to 15.

18. A therapeutic agent for an eye disease comprising the composition according to any one of claims 1 to 15.

19. The therapeutic agent for an eye disease according to claim 18, wherein the eye disease is macular hole or retinal detachment.

20. Atreatment method for an eye disease, comprising a step of administering to a subject a therapeutically effective amount of the composition according to any one of claims 1 to 15.

21. The treatment method according to claim 20, wherein the eye disease is macular hole or retinal detachment.

22. A production method of the composition according to any one of claims 8 to 15, which comprises making the polymerized peptide represented by the formula (I) and the aqueous filler into a homogeneous physical mixture.

23. Use of the composition according to any one of claims 1 to 15 in the production of the pharmaceutical product for treating an eye disease.
